# EUROPEAN PATENT SPECIFICATION

(11) **EP 3 925 605 B1**
(45) Date of publication and mention of the grant of the patent: **21.08.2024**
(21) Application number: 21175048.4
(22) Date of filing: 20.05.2021
(51) Int. Cl.: A61K 31/343, A61P 25/14, A61P 25/22, A61P 37/04, A61P 43/00

(54) **METHOD FOR PREVENTING AND/OR TREATING A STRESS-INDUCED DISEASE**
VERFAHREN ZUR VORBEUGUNG UND/ODER BEHANDLUNG VON STRESSINDUZIERTEN ERKRANKUNGEN
PROCÉDÉ DE PRÉVENTION ET/OU DE TRAITEMENT D'UNE MALADIE INDUITE PAR LE STRESS

(30) Priority: 19.06.2020 US 202016906418
(43) Date of publication of application: 22.12.2021
(73) Proprietor: Tzu Chi University, Hualien City, Hualien County 970 (TW)
(72) Inventor: CHANG, Hsin-Hou, Hualien City, Hualien County 970 (TW); SUN, Der-Shan, Hualien City, Hualien County 970 (TW)
(74) Representative: J A Kemp LLP

(56) References cited:
- WO-A1-2013/111969
- WO-A1-2020/092978
- CN-A- 107 913 277
- US-A1- 2018 228 858
- LU JIAQI ET AL: "Tanshinone IIA Improves Depression-like Behavior in Mice by Activating the ERK-CREB-BDNF Signaling Pathway", NEUROSCIENCE, NEW YORK, NY, US, vol. 430, 31 January 2020 (2020-01-31), pages 1 - 11, XP086066387, ISSN: 0306-4522, [retrieved on 20200131], DOI: 10.1016/J.NEUROSCIENCE.2020.01.026

## Description

### FIELD OF THE INVENTION

The present disclosure relates generally to treating stress-induced diseases. More specifically, the disclosure relates to uses of tanshinone IIA and derivatives thereof in preventing and/or treating stress-induced diseases.

### BACKGROUND OF THE INVENTION

Stress (e.g. psychological stress and physiological stress) is known to induce gastrointestinal leakage (and damage) and immune suppression (Price et al., J Exp Biol 2013; 216:4065-70; Chan et al., Oncotarget 2018; 9:12781-95). Intriguingly, inflammatory pathways play protective roles against gastrointestinal injury (Wang et al., Lab Invest 2013; 93:1024-35). This may indicate that normal levels of inflammatory response are critical for protection and maintenance of gastrointestinal homeostasis, and an excessive anti-inflammatory response may be harmful in certain pathological and stressed conditions. Although the mechanism remains elusive, it has been reported that intestinal abnormalities and leakage are associated with abnormal immunosuppressive regulation.

Tanshinones are a class of chemical compounds, which includes dihydrotanshinone, tanshinone I, tanshinone IIA, tanshinone IIA sodium sulfate, crytotanshinone (cryptotanshinone). These compounds can be isolated from *Salvia miltiorrhiza* (Jiang et al., Front Pharmacol 2019; 10:202). Tanshinone IIA has been reported to have certain anti-inflammatory and immune suppressive effect (Chen et al., Front Pharmacol 2019; 10:850).
Ohlsson et al., (Acta Psychiatr Scand 2019: 139: 185-193) discuss leaky gut biomarkers in depression and suicidal behavior.

### SUMMARY OF THE INVENTION

The invention is defined by the claims. Any subject-matter falling outside the scope of the claims is provided for information purposes only. The present disclosure provides a compound for preventing and/or treating a stress-induced gastrointestinal leakage and/or gastrointestinal injury in a subject in need of such treatment, comprising administering to said subject an effective amount of tanshinone IIA or a derivative thereof, as defined in the claims, or a pharmaceutically acceptable salt, solvate, hydrate, isotopologue.

The derivative of tanshinone IIA is selected from the group consisting of tanshinone I, crytotanshinone (cryptotanshinone), and tanshinone IIA sodium sulfate.

The IUPAC names of tanshinone IIA, tanshinone I, and cryptotanshinone are as follows: Tanshinone IIA: 1,6,6-trimethyl-8,9-dihydro-7H-naphtho[1,2-g][1]benzofuran-10,11-dione Tanshinone I: 1,6-dimethylnaphtho[1,2-g][1]benzofuran-10,11-dione Cryptotanshinone: (1R)-1,6,6-trimethyl-2,7,8,9-tetrahydro-1H-naphtho[1,2-g][1]benzofuran-10,11-dione The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

In one embodiment of the disclosure, the stress-induced disease is a psychological stress-induced disease.

In one embodiment of the disclosure, the stress-induced disease is a physiological stress-induced disease.

In one embodiment of the disclosure, the stress-induced disease is a cold stress-induced disease.

In one embodiment of the disclosure, the stress-induced disease is a restraint stress-induced disease.

In one embodiment of the disclosure, the stress-induced disease is a gastrointestinal injury.

In one embodiment of the disclosure, the stress-induced disease is immunosuppression.

In one embodiment of the disclosure, the immunosuppression is immunosuppressive steroid hormone elevation.

In one embodiment of the disclosure, the immunosuppression is infection.

In one embodiment of the disclosure, the immunosuppression is tissue damage.

The present disclosure provides a compound for use in a method for preventing and/or treating a stress-induced disorder according to the claims in a subject in need of such treatment, wherein the pharmaceutical composition comprises an effective amount of tanshinone IIA or a derivative thereof as claimed, or a pharmaceutically acceptable salt, solvate, hydrate, isotopologue thereof.

The present disclosure is described in detail in the following sections. Other characteristics, purposes and advantages of the present invention can be found in the detailed description and claims. The invention is defined by the claims. Any subject-matter falling outside the scope of the claims is provided for information purposes only.

### BRIEF DESCRIPTION OF THE DRAWINGS

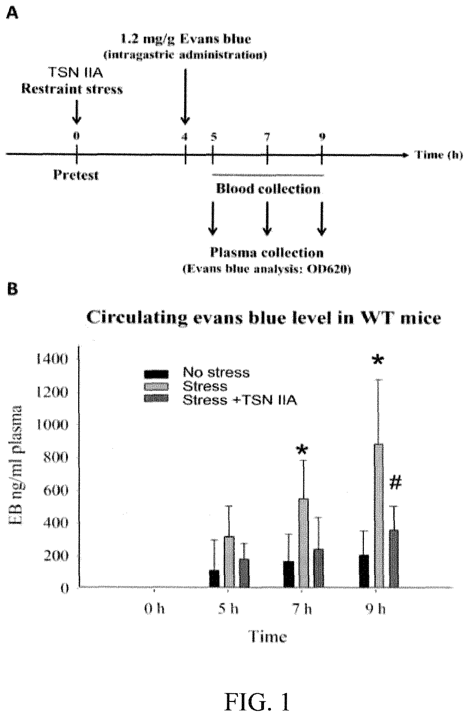

FIG. 1 shows exemplary rescue of restraint stress-induced gastrointestinal leakage by tanshinone IIA (TSNIIA) treatments. Experiment outlines (A). The effects of tanshinone IIA treatments on the amelioration of restraint stress induced gastrointestinal leakage (B) were analyzed. The data are representative of three independent experiments with two mice per group *(n* = 6). * *P* < 0.05 compared with the respective 5h, no stress groups, # *P* < 0.05 compared with the respective stress groups of each time course (B). EB: Evans blue.

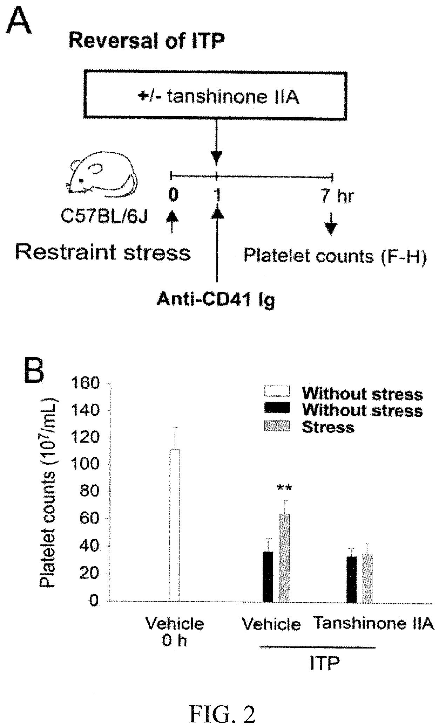

FIG. 2 shows exemplary rescue of restraint stress-mediated immunosuppression by tanshinone IIA treatments. Experiment outlines (A). The effects of tanshinone IIA treatments on the restraint stress-mediated amelioration of immune thrombocytopenia (ITP) (B) were analyzed. The data are representative of three independent experiments with two mice per group *(n* = 6). ** *P* < 0.01 compared with the respective vehicle groups in the tanshinone IIA vs. vehicle treatments (B).

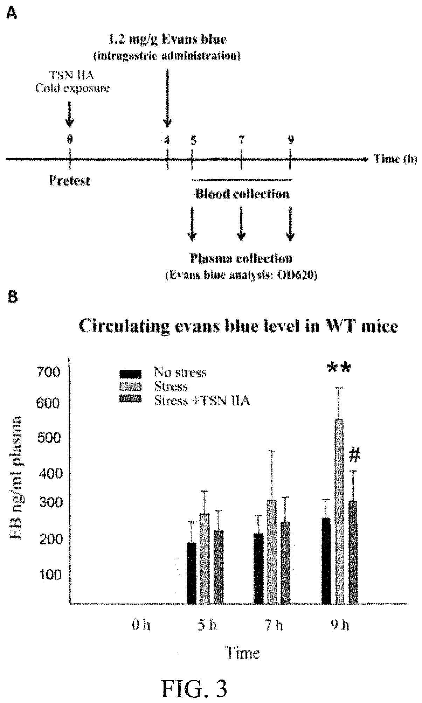

FIG. 3 shows exemplary rescue of cold stress-induced gastrointestinal leakage by tanshinone IIA (TSNIIA) treatments. Experiment outlines (A). The effects of tanshinone IIA treatments on the amelioration of cold exposure-induced gastrointestinal leakage (B) were analyzed. The data are representative of three independent experiments with two mice per group *(n* = 6). ** *P* < 0.01 compared with the respective 5h, no stress groups, # *P* < 0.05 compared with the respective stress groups of each time course (B). EB: Evans blue.

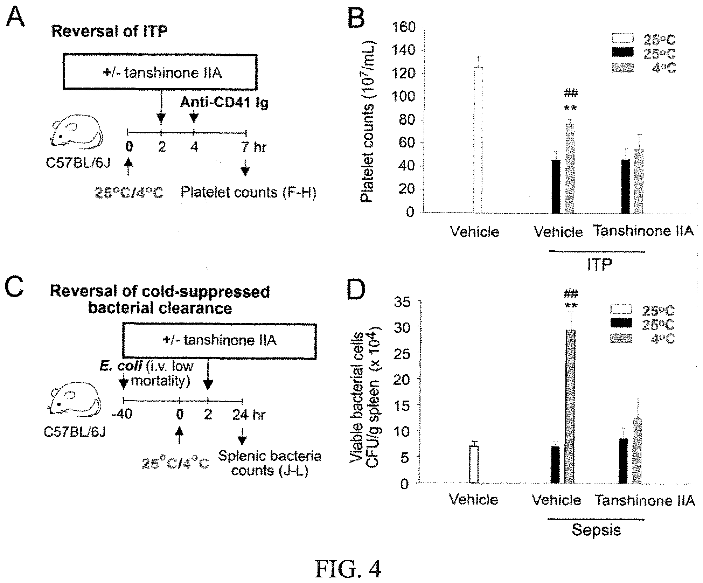

FIG. 4 shows exemplary rescue of cold stress-mediated immunosuppression by tanshinone IIA treatments. Experiment outlines (A and C). The effects of tanshinone IIA treatments on the cold-mediated amelioration of immune thrombocytopenia (ITP) (B) and cold-suppressed bacterial clearance (D) were analyzed. The data are representative of three independent experiments with two mice per group *(n* = 6). ** *P* < 0.01 compared with the respective vehicle (25° C) groups, ## *P* < 0.01 compared with the respective tanshinone IIA 25° C groups (B and D).

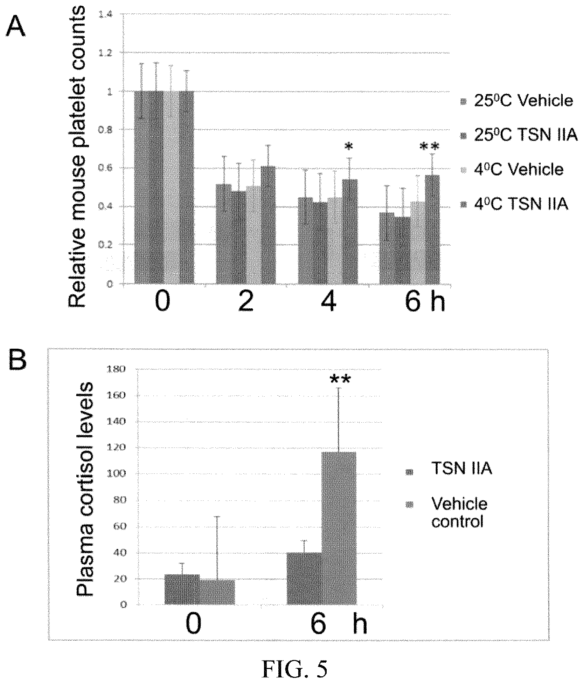

FIG. 5 shows exemplary rescue of cold stress-induced immunosuppression and plasma cortisol levels by tanshinone IIA treatments. The time course on the effects of tanshinone IIA treatments on the cold-mediated amelioration of immune thrombocytopenia (ITP) (A) and cold-induced elevation of cortisol (B) was analyzed. The data are representative of three independent experiments with two mice per group *(n* = 6). ** *P* < 0.01 compared with the respective vehicle (25 °C)/vehicle (0 h) groups.

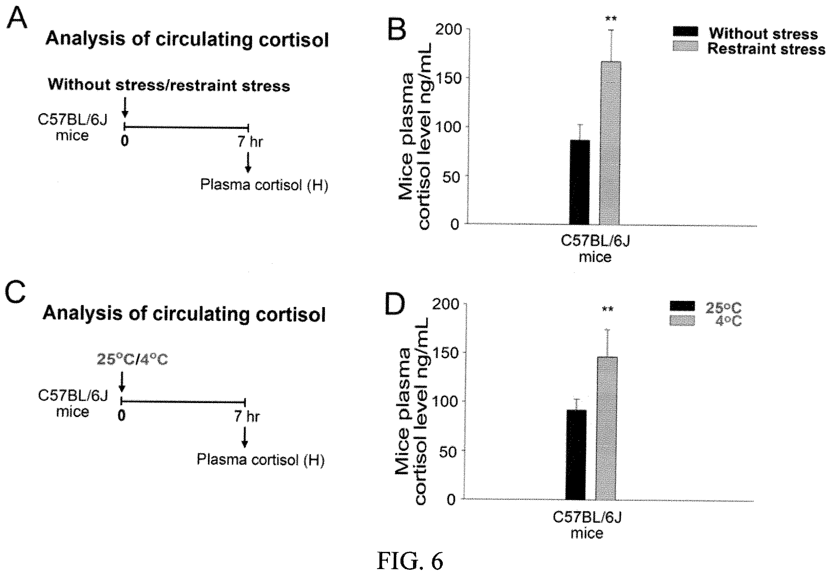

FIG. 6 shows elicitation of circulating cortisol levels under restraint stress and cold stress. The plasma cortisol levels of experimental mice under restraint stress and cold stress were analyzed using ELISA. The data are representative of two independent experiments with two mice per group *(n* = 4). * *P* < 0.05 compared with the respective vehicle groups.

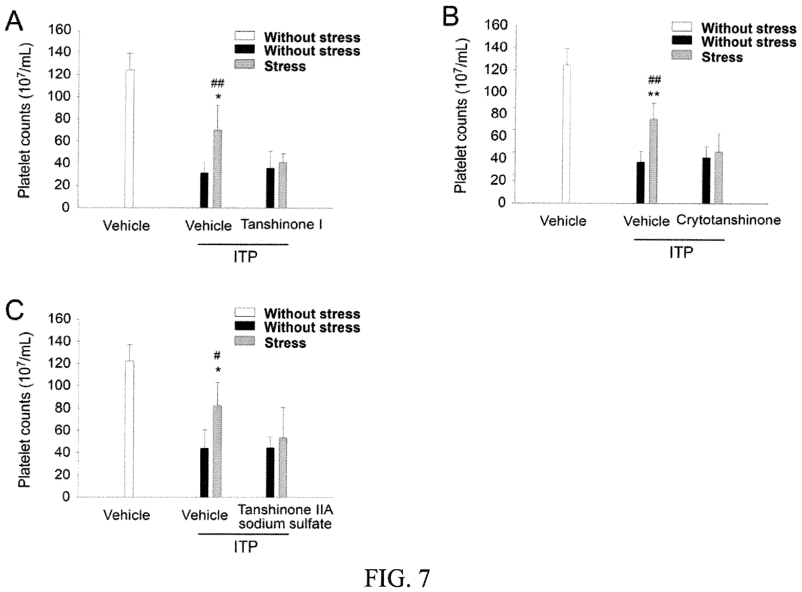

FIG. 7 shows exemplary rescue of restraint stress-mediated immunosuppression by tanshinone IIA derivatives treatments. The effects of tanshinone I (A), crytotanshinone (cryptotanshinone) (B), tanshinone IIA sodium sulfate (C) treatments on the restraint stress -mediated amelioration of immune thrombocytopenia (ITP) were analyzed. The data are representative of three independent experiments with two mice per group *(n* = 6). * *P* < 0.05, ** *P* < 0.01 compared with the respective vehicle without stress (0 h) groups, # *P* < 0.05, ## *P* < 0.01 compared with the respective tanshinone IIA, without stress groups in the ITP experiments.

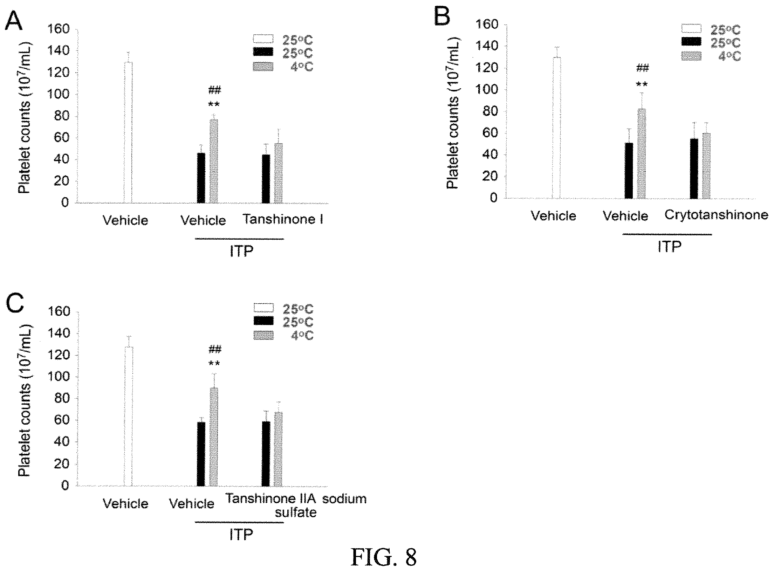

FIG. 8 shows exemplary rescue of cold stress-mediated immunosuppression by tanshinone IIA derivatives treatments. The effects of tanshinone I (A), crytotanshinone (cryptotanshinone) (B), tanshinone IIA sodium sulfate (C) treatments on the cold-mediated amelioration of immune thrombocytopenia (ITP) were analyzed. The data are representative of three independent experiments with two mice per group *(n* = 6). ** *P* < 0.01 compared with the respective vehicle (25° C) groups, ## *P* < 0.01 compared with the respective tanshinone IIA 25° C groups.

### DETAILED DESCRIPTION OF THE INVENTION

The present invention can be more readily understood by reference to the following detailed description of various embodiments of the invention, and the examples.

As utilized in accordance with the present disclosure, the following terms, unless otherwise indicated, shall be understood to have the following meaning:
It must be noted that, as used in the specification and the appended claims, the singular forms "a," "an" and "the" include plural referents unless the context clearly dictates otherwise. Thus, unless otherwise required by context, singular terms shall include the plural and plural terms shall include the singular.

As used herein, the term "or" is used to mean "and/or" unless explicitly indicated to refer to alternatives only or the alternatives are mutually exclusive.

As used herein, the terms "subject" and "patient" are used interchangeably herein and will be understood to refer to a warm blooded animal, particularly a mammal. Non-limiting examples of animals within the scope and meaning of this term include guinea pigs, dogs, cats, rats, mice, horses, goats, cattle, sheep, zoo animals, non-human primates, and humans.

The term "effective amount" of an active ingredient as provided herein means a sufficient amount of the ingredient to provide the desired regulation of a desired function. As will be pointed out below, the exact amount required will vary from subject to subject, depending on the disease state, physical conditions, age, sex, species and weight of the subject, the specific identity and formulation of the composition, etc. Dosage regimens may be adjusted to induce the optimum therapeutic response. For example, several divided doses may be administered daily or the dose may be proportionally reduced as indicated by the exigencies of the therapeutic situation. Thus, it is not possible to specify an exact "effective amount." However, an appropriate effective amount can be determined by one of ordinary skill in the art using only routine experimentation.

The term "preventing" or "prevention" is recognized in the art, and when used in relation to a condition, it includes administering, prior to onset of the condition, an agent to reduce the frequency or severity of or delay the onset of symptoms of a medical condition in a subject, relative to a subject which does not receive the agent.

The term "treating" or "treatment" as used herein denotes reversing, alleviating, inhibiting the progress of, or improving the disorder, disease or condition to which such term applies, or one or more symptoms of such disorder, disease or condition.

"Optional" or "optionally" means that the subsequently described event or circumstance may or may not occur, and that the description includes instances where said event or circumstance occurs and instances where it does not. For example, the phrase "optionally comprising an agent" means that the agent may or may not exist.

The term "a pharmaceutically acceptable derivative" or "pharmaceutically acceptable derivatives" as used herein denotes a compound that is modified from the compound of the invention but has properties and efficacies that are the same as or better than those of the compound of the invention. Preferably, the pharmaceutically acceptable derivative is a pharmaceutically acceptable salt, solvate, hydrate, isotopologue, or prodrug of the compound of the invention.

The term "carrier" or "excipient" as used herein refers to any substance, not itself a therapeutic agent, used as a carrier and/or diluent and/or adjuvant, or vehicle for delivery of a therapeutic agent to a subject or added to a formulation to improve its handling or storage properties or to permit or facilitate formation of a dose unit of the composition into a discrete article such as a capsule or tablet suitable for oral administration. Suitable carriers or excipients are well known to persons of ordinary skill in the art of manufacturing pharmaceutical formulations or food products. Carriers or excipients can include, by way of illustration and not limitation, buffers, diluents, disintegrants, binding agents, adhesives, wetting agents, polymers, lubricants, glidants, substances added to mask or counteract a disagreeable taste or odor, flavors, dyes, fragrances, and substances added to improve the appearance of the composition. Acceptable carriers or excipients include citrate buffer, phosphate buffer, acetate buffer, bicarbonate buffer, stearic acid, magnesium stearate, magnesium oxide, sodium and calcium salts of phosphoric and sulfuric acids, magnesium carbonate, talc, gelatin, acacia gum, sodium alginate, pectin, dextrin, mannitol, sorbitol, lactose, sucrose, starches, gelatin, cellulosic materials (such as cellulose esters of alkanoic acids and cellulose alkyl esters), low melting wax cocoa butter, amino acids, urea, alcohols, ascorbic acid, phospholipids, proteins (for example, serum albumin), ethylenediamine tetraacetic acid (EDTA), dimethyl sulfoxide (DMSO), sodium chloride or other salts, liposomes, mannitol, sorbitol, glycerol or powder, polymers (such as polyvinyl-pyrrolidone, polyvinyl alcohol, and polyethylene glycols), and other pharmaceutically acceptable materials. The carrier should not destroy the pharmacological activity of the therapeutic agent and should be non-toxic when administered in doses sufficient to deliver a therapeutic amount of the agent.

In contrast to the known anti-inflammatory and immune suppressive effect of tanshinone IIA, the present disclosure demonstrates for the first time that tanshinone IIA and derivatives thereof are able to prevent and/or treat a stress-induced disease associated with increasing immune responses and counteracting immunosuppression. In addition, treatments of tanshinone IIA and its derivatives have ameliorative effects on stress-associated intestinal leakage and damage.

Accordingly, the present disclosure describes a method for preventing and/or treating a stress-induced disease in a subject in need of such treatment, comprising administering to said subject an effective amount of tanshinone IIA or a derivative thereof, or a pharmaceutically acceptable salt, solvate, hydrate, isotopologue, or prodrug of tanshinone IIA or a derivative of tanshinone IIA.

The derivative of tanshinone IIA is selected from the group consisting of tanshinone I, crytotanshinone (cryptotanshinone), and tanshinone IIA sodium sulfate.

Tanshinone IIA and its derivatives can also exist as a solvate or hydrate. Thus, these compounds may crystallize with, for example, waters of hydration, or one, a number of, or any fraction of molecules of the mother liquor solvent. The solvates and hydrates of such compounds are included within the scope of this invention.

Tanshinone IIA or a derivative of tanshinone IIA, or a pharmaceutically acceptable salt, solvate, hydrate, isotopologue, is preferably comprised in a pharmaceutical composition or food composition, according to the disclosure.

The tanshinone IIA or a derivative of tanshinone IIA or a pharmaceutical composition according to the disclosure is preferably administered topically or systemically by any method known in the art, including, but not limited to, intramuscular, intradermal, intravenous, subcutaneous, intraperitoneal, intranasal, oral, mucosal or external routes. The appropriate route, formulation and administration schedule can be determined by those skilled in the art. In the present disclosure, the pharmaceutical composition can be formulated in various ways, according to the corresponding route of administration, such as a liquid solution, a suspension, an emulsion, a syrup, a tablet, a pill, a capsule, a sustained release formulation, a powder, a granule, an ampoule, an injection, an infusion, a kit, an ointment, a lotion, a liniment, a cream or a combination thereof. If necessary, it may be sterilized or mixed with any pharmaceutically acceptable carrier or excipient, many of which are known to one of ordinary skill in the art.

The external route as used herein is also known as local administration, which includes but is not limited to administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulisation, suppositories, pessaries, retention enemas and chewable or suckable tablets or pellets or liposome or microencapsulation preparations.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilizing agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilizing agents, suspending agents or preservatives.

Spray compositions may, for example, be formulated as aqueous solutions or suspensions or as aerosols delivered from pressurized packs, such as a metered dose inhaler, with the use of a suitable liquefied propellant. Aerosol compositions suitable for inhalation can be either a suspension or a solution. The aerosol composition may optionally contain additional formulation excipients well known in the art such as surfactants, e.g., oleic acid or lecithin and cosolvents, e.g., ethanol.

Topical preparations may be administered by one or more applications per day to the affected area; over the skin area occlusive dressings may advantageously be used. Continuous or prolonged delivery may be achieved by an adhesive reservoir system.

Tanshinone IIA or a derivative of tanshinone IIA, or a pharmaceutically acceptable salt, solvate, hydrate, isotopologue, can be added to a conventional food composition (i.e., the edible food or drink or precursors thereof) in the manufacturing process of the food composition. Almost all food compositions can be supplemented with the extract composition of the invention. The food compositions that can be supplemented with the extract composition of the invention include, but are not limited to, candies, baked goods, ice creams, dairy products, sweet and flavor snacks, snack bars, meal replacement products, fast foods, soups, pastas, noodles, canned foods, frozen foods, dried foods, refrigerated foods, oils and fats, baby foods, or soft foods painted on breads, or mixtures thereof.

The stress-induced disease includes a psychological stress-induced disease and a physiological stress-induced disease. In one embodiment, the stress-induced disease is a cold stress-induced disease, which is the physiological stress-induced disease. In another aspect, the stress-induced disease is a restraint stress-induced disease, which is the psychological stress-induced disease. In still another aspect, the stress-induced disease is a gastrointestinal injury. In still another aspect, the stress-induced disease is immunosuppression.

In one embodiment of the disclosure, the immunosuppression is immunosuppressive steroid hormone elevation, infection or tissue damage.

In one embodiment of the disclosure, it is found that treatments of tanshinone IIA and its derivatives can increase the immune response and counteract the immunosuppression associated with the stress. In addition, treatments of tanshinone IIA and its derivatives have ameliorative effects on stress-associated intestinal leakage and damage. As tanshinone IIA treatments can ameliorate cold stress-induced levels of circulating steroid hormone, and both restraint stress and cold stress induce elevation of circulating steroid hormone, the immune enhancing effect of tanshinone IIA is likely in part mediated through the suppression of such anti-inflammatory and immunosuppressive steroid hormone. Collectively, these results show that tanshinone IIA and its derivatives are gastrointestinal protective and immune enhancing agents to achieve homeostasis under stress.

The present disclosure is to provide use of a pharmaceutical composition in the manufacture of a medicament for preventing and/or treating a stress-induced disease in a subject in need of such treatment, wherein the pharmaceutical composition comprises an effective amount of tanshinone IIA or a derivative of tanshinone IIA, or a pharmaceutically acceptable salt, solvate, hydrate, isotopologue, and optionally a pharmaceutically acceptable carrier or excipient.

The present disclosure is to provide a pharmaceutical composition for use in preventing and/or treating a stress-induced disease in a subject in need of such treatment, wherein the pharmaceutical composition comprises an effective amount of tanshinone IIA or a derivative of tanshinone IIA as claimed, or a pharmaceutically acceptable salt, solvate, hydrate, isotopologue, and optionally a pharmaceutically acceptable carrier or excipient.

The following examples are provided to aid those skilled in the art in practicing the present invention.

### EXAMPLES

### Methods

### Induction and rescue of inflammation and ITP

Autoimmune thrombocytopenia (ITP) mouse model, through intravascular injections of anti-platelet (CD41) Ig following previously reported methods (Huang et al., Blood 2010; 116:5002-9), was employed to investigate the immunosuppressive property of cold exposure and restraint stress, and immune enhancing properties of tanshinone IIA and its derivatives. The B57BL/6J mice were intravenously injected with 0.1 mg/kg (body weight) of an antiplatelet monoclonal antibody (rat antimouse integrin αIIb/CD41 Ig, clone MWReg30, BD PharMingen) to induce ITP. Mice with or without injections of anti-CD41 Ig were subjected to cold exposure and restraint stress. To address the immune enhancing properties of tanshinone IIA, these mice were further treated with or without tanshinone I, tanshinone IIA, tanshinone IIA sodium sulfate, crytotanshinone (cryptotanshinone) (10-2 to 10 mg/kg) treatments. To determine the mouse platelet counts, whole blood samples (100-120 µL) of the mice were collected from the retro-orbital venous plexus and mixed with an anticoagulant, a citrate dextrose solution (38 mM citric acid, 75 mM sodium citrate, and 100 mM dextrose), in Eppendorf tubes. Subsequently, platelet counts were measured using a hematology analyzer (KX-21N, Sysmex) at various time intervals as previously described (Chan et al., Oncotarget 2018; 9:12781-95; Huang et al., Blood 2010; 116:5002-9).

### Bacterial clearance and sepsis-induced mortality under stress

The bacterium *E. coli* was cultured using standard methods. To analyze the immunosuppressive effects of cold exposure and restraint stress on bacterial clearance, following our previously developed methods (Chan et al., Oncotarget 2018; 9:12781-95), the experimental mice were challenged by intravenously administering bacteria (*E. coli,* BL21, 6 × 10⁹ CFU/kg; no mortality within 24 h at 4 °C); a 40-h circulating equilibrium at a 25° C environment was required before cold exposure (control groups: 25° C exposure) and restraint stress. One day after treatment with cold exposure or restraint stress, the spleens of the euthanized mice were collected and weighed. After homogenization (homogenizer, *BioSpec Products, Racine, WI, USA*) in PBS, surviving bacteria (colony forming units [CFUs]) in the spleen tissues were quantified using the standard plating method. Tanshinone I, tanshinone IIA, tanshinone IIA sodium sulfate, crytotanshinone (cryptotanshinone) (10⁻² to 10 mg/kg) treatments were used to rescue cold-induced and restraint stress immunosuppression; all reagents were treated through a feeding tube, 2 h after cold exposure and restraint stress.

### Measurements of cortisol

Blood samples were collected from experimental mice under cold stress or restraint stress for 7 h. The plasma levels of cortisol were measured using an ELISA kit (Cayman Chemical, Ann Arbor, MI, USA) as previously described (Chan et al., Oncotarget 2018; 9:12781-95).

### Results

The results of the exemplary rescue of restraint stress-induced gastrointestinal leakage by tanshinone IIA (TSNIIA) treatments are shown in FIG. 1. Evans blue, a gastrointestinal non-absorbable dye, is able to bind circulating albumin to form dye-protein complexes, which cannot normally penetrate the endothelial cell layers of the vascular system, unless the tissue has been damaged (Yao et al., Contrast Media Mol Imaging 2018; 2018:7628037). Accordingly, circulating Evens blue levels are approximately and proportionally correlated to the levels of gastrointestinal injury. By comparing the stressed, 9h groups, with or without treatments of TSNIIA, we can find that TSNIIA is able to ameliorate stress-induced gastrointestinal injury in B57BL/6J mice (# *P* < 0.05).

The results of the exemplary rescue of restraint stress-mediated immunosuppression by tanshinone IIA treatments are shown in FIG. 2. The statistical analysis revealed that after restraint stress, the immunosuppression suppressed the anti-platelet (CD41) Ig induced thrombocytopenia (platelet count become higher, ** *P* < 0.01, B) in mice. By contrast, the tanshinone IIA treatments ameliorate the immunosuppressive effect, and therefore the platelet counts in the tanshinone IIA-treated groups remain low and do not show statistical significance as compared to the vehicle control (without immunosuppression) groups.

The results of the exemplary rescue of cold stress-induced gastrointestinal leakage by tanshinone IIA (TSNIIA) treatments are shown in FIG. 3. By comparing the stressed, 9h groups, with or without treatments of TSNIIA, we can find that TSNIIA is able to ameliorate cold stress-induced gastrointestinal injury in B57BL/6J mice (# *P* < 0.05).

The result of the exemplary rescue of cold stress-mediated immunosuppression by tanshinone IIA treatments are shown in FIG. 4. The statistical analysis revealed that after the cold stress, the immunosuppression suppressed the anti-platelet (CD41) Ig induced thrombocytopenia (platelet count became higher, ** *P* < 0.01, B) and bacterial clearance rate (higher bacteria survival, ** *P* < 0.01, D) in mice. By contrast, the tanshinone IIA treatments ameliorate the immunosuppressive effect, and therefore the platelet counts in the tanshinone IIA-treated groups remain low and do not show statistical significance as compared to the vehicle control (without immunosuppression) groups (B). Similarly, because tanshinone IIA treatments ameliorate the immunosuppressive effect, the bacterial clearance rate is high and tanshinone IIA treated groups do not show statistical significance as compared to the vehicle control (without immunosuppression) groups (D).

The results of the exemplary rescue of cold stress-induced immunosuppression and plasma cortisol levels by tanshinone IIA treatments are shown in FIG. 5. These results show that tanshinone IIA treatments counteract the cold stress-induced levels of circulating anti-inflammatory steroid hormone cortisol in mice. Because anti-inflammatory steroid hormone levels are suppressed, this could be one of the mechanisms underlining tanshinone IIA-mediated restoration and enhancement of the immune system.

The results of the elicitation of circulating cortisol levels under restraint stress and cold stress are shown in FIG. 6. These results show that the psychological (restraint stress) and physiological (cold) stress described herein are a type of stress associated with steroid hormone elicitation.

The results of the exemplary rescue of restraint stress-mediated immunosuppression by tanshinone IIA derivatives treatments are shown in FIG. 7. These results show that tanshinone IIA and its derivatives of tanshinone I, crytotanshinone (cryptotanshinone), tanshinone IIA sodium sulfate exert similar immune enhancing properties under psychological stress.

The results of the exemplary rescue of cold stress-mediated immunosuppression by tanshinone IIA derivatives treatments are shown in FIG. 8. These results show that tanshinone IIA and its derivatives of tanshinone I, crytotanshinone (cryptotanshinone), tanshinone IIA sodium sulfate exert similar immune enhancing properties under cold stress.

## Claims

1. A compound which is tanshinone IIA or a derivative thereof, or a pharmaceutically acceptable salt, solvate, hydrate, or isotopologue of tanshinone IIA, for use in preventing and/or treating a stress-induced gastrointestinal leakage and/or gastrointestinal injury in a subject in need of such treatment, wherein the derivative of tanshinone IIA is selected from the group consisting of tanshinone I, crytotanshinone (cryptotanshinone), and tanshinone IIA sodium sulfate.

2. The compound for use according to claim 1, wherein the stress-induced gastrointestinal leakage and/or gastrointestinal injury is a psychological stress-induced gastrointestinal leakage and/or gastrointestinal injury.

3. The compound for use according to claim 1, wherein the stress-induced gastrointestinal leakage and/or gastrointestinal injury is a physiological stress-induced gastrointestinal leakage and/or gastrointestinal injury.

4. The compound for use according to claim 1, wherein the stress-induced gastrointestinal leakage and/or gastrointestinal injury is a cold stress-induced gastrointestinal leakage and/or gastrointestinal injury.

5. The compound for use according to claim 1, wherein the stress-induced gastrointestinal leakage and/or gastrointestinal injury is a restraint stress-induced gastrointestinal leakage and/or gastrointestinal injury.

## Patentansprüche

1. Verbindung, die Tanshinon IIA oder ein Derivat davon ist, oder ein pharmazeutisch unbedenkliches Salz, Solvat, Hydrat oder Isotopolog von Tanshinon IIA, für die Verwendung zum Verhindern und/oder Behandeln einer stressinduzierten gastrointestinalen Leckage und/oder gastrointestinalen Verletzung bei einem Subjekt, das einer solchen Behandlung bedarf, wobei das Derivat von Tanshinon IIA aus der Gruppe ausgewählt ist, bestehend aus Tanshinon I, Crytotanshinon (Cryptotanshinon) und Tanshinon-IIA-Natriumsulfat.

2. Verbindung für die Verwendung nach Anspruch 1, wobei die stressinduzierte gastrointestinale Leckage und/oder gastrointestinale Verletzung eine psychologische, stressinduzierte gastrointestinale Leckage und/oder gastrointestinale Verletzung ist.

3. Verbindung für die Verwendung nach Anspruch 1, wobei die stressinduzierte gastrointestinale Leckage und/oder gastrointestinale Verletzung eine physiologische stressinduzierte gastrointestinale Leckage und/oder gastrointestinale Verletzung ist.

4. Verbindung für die Verwendung nach Anspruch 1, wobei die stressinduzierte gastrointestinale Leckage und/oder gastrointestinale Verletzung eine durch Kältestress induzierte gastrointestinale Leckage und/oder gastrointestinale Verletzung ist.

5. Verbindung für die Verwendung nach Anspruch 1, wobei die stressinduzierte gastrointestinale Leckage und/oder gastrointestinale Verletzung eine durch Fesselungsstress induzierte gastrointestinale Leckage und/oder gastrointestinale Verletzung ist.

## Revendications

1. Composé qui est la tanshinone IIA ou un dérivé de celle-ci, ou un sel, solvate, hydrate, ou isotopologue pharmaceutiquement acceptable de tanshinone IIA, destiné à être utilisé pour prévenir et/ou traiter une fuite gastro-intestinale et/ou une lésion gastro-intestinale induites par le stress chez un sujet nécessitant un tel traitement, dans lequel le dérivé de tanshinone IIA est sélectionné dans le groupe constitué par la tanshinone I, la crytotanshinone (cryptotanshinone), et le sulfate sodique de tanshinone IIA.

2. Composé destiné à être utilisé selon la revendication 1, dans lequel la fuite gastro-intestinale et/ou la lésion gastro-intestinale induites par le stress sont une fuite gastro-intestinale et/ou une lésion gastro-intestinale induites par le stress psychologique.

3. Composé destiné à être utilisé selon la revendication 1, dans lequel la fuite gastro-intestinale et/ou la lésion gastro-intestinale induites par le stress sont une fuite gastro-intestinale et/ou une lésion gastro-intestinale induites par le stress physiologique.

4. Composé destiné à être utilisé selon la revendication 1, dans lequel la fuite gastro-intestinale et/ou la lésion gastro-intestinale induites par le stress sont une fuite gastro-intestinale et/ou une lésion gastro-intestinale induites par le stress dû au froid.

5. Composé destiné à être utilisé selon la revendication 1, dans lequel la fuite gastro-intestinale et/ou la lésion gastro-intestinale induites par le stress sont une fuite gastro-intestinale et/ou une lésion gastro-intestinale induites par le stress de contention.
